# EUROPEAN PATENT APPLICATION

(11) **EP 4 268 729 A1**
(43) Date of publication of application: **01.11.2023**
(21) Application number: 21863039.0
(22) Date of filing: 22.12.2021
(51) Int. Cl.: A61B 8/08, G06T 7/00

(54) **METHOD FOR THE AUTOMATED ASSESSMENT OF LUNG ULTRASOUND SCANS AND ULTRASOUND MACHINE WHICH IMPLEMENTS SAID METHOD**

(30) Priority: 22.12.2020 ES 202031284
(71) Applicant: Consejo Superior De Investigaciones Científicas (CSIC), 28006 Madrid (ES)
(72) Inventor: CAMACHO SOSA-DIAS, Jorge, 28006 Madrid (ES); GÓMEZ ÁLVAREZ-ARENAS, Tomás, 28006 Madrid (ES); GENOVÉS GÓMEZ, Vicente, 28006 Madrid (ES); TUNG CHEN, Yale, 28222 Majadahonda - Madrid (ES)
(74) Representative: Pons
(86) International application number: PCT/ES2021/070926
(87) International publication number: WO 2022/136720

(57) **Abstract**

The present invention relates to a method for the automated assessment of lung ultrasound scans, detecting the typical artifacts in patients with pneumonia and showing them, comprising the phases of detecting the pleura; detecting A lines, as lines with a morphology similar to the pleura located at twice the depth; detecting B lines, obtaining a slope value and comparing it with the expected slope for a nominal attenuation of the medium, the lines of low attenuation being B lines; detecting a pleural irregularity, measuring the distance between 6 decibel drop points around the pleura and comparing it with a threshold; detecting a pleural effusion, calculating the mean value of the image between the pleura and a local maximum located immediately above the pleura, and comparing it with a threshold; and calculating a degree of affectation, as the percentage of lines in the image affected by artifacts with respect to the total number of lines that pass through the pleura.

## Description

### OBJECT OF THE INVENTION

The present invention relates to a method for the automated assessment of lung ultrasound scans, automatically identifying and quantifying artifacts related to pathologies and present in the images generated by an ultrasound machine.

An object of the invention is the method for the automated assessment of lung ultrasound scans, which enables the automated calculation of the degree of affectation in lung ultrasound scans and the visual representation of the artifacts that are related to said degree of affectation.

Another object of the invention is an ultrasound machine that implements the method for the automated assessment of lung ultrasound scans and enables an analysis of said lung ultrasound scans to be carried out by non-specialised personnel.

### BACKGROUND OF THE INVENTION

Lung ultrasound scans are based on emitting an ultrasonic pulse (acoustic wave) that travels through the tissue from the skin into the chest. In a normal lung, the air present in the lung parenchyma reflects almost all the energy of the incident wave (high acoustic impedance mismatch between the tissue and the air), so that in the image the pleura is distinguished as a bright line and successive sound reverberations are distinguished between the probe and the pleura, artifacts known as A lines. In this case, a very low percentage of the wave gets into the lung parenchyma, and the signal is quickly attenuated below the pleura.

In a patient with pneumonia, the air in the lung parenchyma is replaced by interstitial fluid or tissue, which has less impedance mismatch with respect to the muscle tissue and the pleura. Therefore, a greater amount of acoustic energy manages to enter the lung parenchyma, and generates a bright line in the image, called B Line. In addition, the width of the echo generated by the pleura increases, which is known as pleural irregularity or thickening, and together with the presence of B lines it is considered pathological. It should be noted that to consider a bright line as a B (pathological) line, A lines, which appear when the lung is normal, must not be present. Another typical ultrasound finding in pneumonia is pleural effusion, when the area between the chest wall and the pleura is infiltrated with fluid. In ultrasound images, it is distinguished by an anechoic area above the pleura and a vertical movement of the latter during breathing.

Lung ultrasound scans are a highly valuable technique for the diagnosis and monitoring of various pathologies, such as pneumonia, pneumothorax, and respiratory distress. In recent months, it has become a fundamental tool to face the COVID-19 pandemic, the main complication of which is pneumonia. Although chest radiography is the most widespread imaging method for the diagnosis of this disease, it has low detection sensitivity for interstitial anomalies, which results in a false negative rate close to 40% in the early stages of the disease.

On the other hand, lung ultrasound scans are a harmless technique (they do not use ionising radiation); they can be performed at the patient's bedside, even with unstable patients, or at primary care or home care centres. The presence of subpleural consolidations, thickened pleural lines, and B lines are highly specific for interstitial syndrome and in these cases they suggest the presence of COVID-19 pneumonia with greater sensitivity than chest radiography and slightly less sensitivity than computed tomography.

However, lung ultrasound scans are still not a very widespread technique. It is normally performed by doctors specialised in ultrasound scanning in emergency or intensive care services, but not in primary care, home care or as a follow-up tool in hospital admissions. The reason is that lung ultrasound scans are difficult to interpret, because they are based on identifying and quantifying artifacts caused by the presence of air in the lungs, and not on more easily interpretable anatomical information.

There is a consensus that the lung ultrasound scan at the patient's bedside is a very valuable tool for diagnosis and follow-up in patients with COVID-19, as it is safe, very specific and can be performed at the patient's bedside. However, it is a technique that is still not widely used, mainly due to the complex interpretation of lung images and the lack of personnel with specific training. Furthermore, in the context of a pandemic, the ultrasound scan with conventional equipment entails a greater risk of contagion for the examiner than other techniques such as chest radiography and computed tomography, for which it would be essential to minimise the duration of the examination and simplify the performance thereof.

WO2014207611A1 discloses a system that employs a lung identification algorithm intended to identify a boundary between heart tissue and lung tissue, on ultrasound imaging. The identification is used to place the cardiac ultrasound probe.

US20140316266A1 discloses an ultrasound-based system to determine the dynamic deformation of a surface by using ultrasound speckle analysis that allows for accurate measurement of surface displacement. The system additionally determines an approximate displacement along a third axis, the z-axis. From these, an estimation of a local volume change is determined. In this way, a volume change can be determined for the lung or any other region of interest.

### DESCRIPTION OF THE INVENTION

The object of this invention is to provide health services with a method and a device capable of analysing lung ultrasound scans in a completely automatic way, identifying and quantifying artifacts present in the image and indicative of interstitial syndrome.

In this way, the application of lung ultrasound scans is to be simplified and made accessible to healthcare personnel without specific training in ultrasound scans, since the method of the invention enables the degree of affectation to be automatically calculated from the ultrasound scan performed. In this way, through interactive image processing tools that simplify the performance of the examination and the interpretation of the images, lung ultrasound scans are to be extended to a greater number of professionals and services, from primary care to intensive care.

By means of the method of the invention, the typical artifacts present in patients with pneumonia (A Lines, B Lines, pleural irregularity and effusion) are automatically detected in real time and visual aids are shown to the examiner on the screen for the detection of said artifacts. In addition, the degree of affectation of the pleura is quantified, presenting a summary of the findings.

The method of the invention can be incorporated into existing ultrasound machines, since it operates based on an imaging sequence generated by the ultrasound machine. In another implementation, the method can be implemented on a computer and worked on imaging sequences previously acquired with an independent ultrasound machine and saved in a standard format.

The method of the invention comprises a step of applying a method for analysing a set of images of the thoracic cavity, obtained by means of a transducer. Said analysis method comprises the phases of detecting the pleura, detecting A lines, detecting B lines, detecting a pleural irregularity and detecting a pleural effusion.

The pleura is identified as a line that separates an upper area from a lower area. The upper area has low variability between the set of images, previously obtained by an ultrasound machine, and corresponds to the muscle tissue. On the other hand, the lower area has high variability between the set of images and corresponds to the lung parenchyma. Preferably, an average of the set of images is used in this phase, wherein a subtraction of consecutive images and a high-pass filter are used to detect the distance at which the pleura is located, which coincides with the position of the maximum at the outlet of said filter.

The detection of A lines is carried out by detecting lines with a morphology similar to the pleura and located at approximately twice the depth in the set of images. To obtain them, the maximum grey value in a region located at twice the distance between the transducer and the pleura is calculated for each line in the image. Said maximum is compared with the mean value and the standard deviation of the grey level in the same region, such that an A line is considered to exist when the quotient between the maximum and the standard deviation is greater than a predefined threshold between 5 and 10. Finally, the vertical distance between the points detected in consecutive lines is calculated and the A line is determined as the set of consecutive points, the distance of which is less than a pre-established threshold between 3 and 5 millimetres.

The B lines are detected by applying a linear regression in the amplitude-depth function of each line in the image, thus obtaining a slope value for each one, and said slope is compared with the expected slope due to a nominal attenuation of the medium, such that those having a low attenuation are identified as B lines. The attenuation of the medium is defined as the signal loss in decibels per centimetre and per megahertz.

The detection of a pleural irregularity is carried out by measuring the width of the echo generated by the pleura, i.e., by measuring the distance between the points where the signal falls 6 dB with respect to the value in the pleura and comparing it with a pre-established threshold which is preferably greater than 1.5 times a period of the ultrasonic signal used to generate the images.

Likewise, the method of the invention may comprise the detection of a pleural effusion, which is carried out by analysing the region immediately above the detected pleura. If it has a well-defined dark area that is observed in several consecutive frames, it is identified as a pleural effusion. To do this, the first peak before the pleura that exceeds a certain value, typically between 75% and 100% of the grey level of the pleura, is sought in each line in the image. The mean value of the image between this peak and the pleura is then calculated and compared with a second threshold, typically between 25% and 50% of the grey level of the pleura, to determine whether or not it is a pleural effusion.

The phases of detecting A lines, detecting B lines, detecting a pleural irregularity, and detecting a pleural effusion can be performed simultaneously or in a tiered manner.

Then a degree of affectation is calculated, such as the percentage of lines in the image affected by B lines, pleural irregularity or pleural effusion with respect to the total number of lines in the image that pass through the pleura. For this, two-dimensional filters can be used to eliminate noise and reduce false positives.

Additionally, the method of the invention may further comprise a previous step of classifying the images obtained as valid or invalid by means of a method for analysing the set of images of the thoracic cavity that determines the variability between images obtained consecutively in an upper area, located above the pleura, such that if said variability is less than a pre-established threshold, the images are considered valid.

Likewise, the method of the invention may also comprise a step of generating a condensed representation of the pleura by calculating the mean value of amplitude below the pleura for each line in the image and constructing the condensed representation by placing the acquisition time on the vertical axis, the horizontal position of each line on the horizontal axis and the mean value of the amplitude coded in grey scale.

Furthermore, the detected artifacts can be highlighted in the original image, by means of an overlay of the A lines, B lines, pleural irregularities and effusions detected on the original image and an overlay of regions affected by A lines, B lines, pleural irregularities and effusions on the condensed representation of the pleural image.

Another object of the invention is an ultrasound machine aimed at the early detection and follow-up of diseases, such as the COVID-19 disease, which enables lung ultrasound scans to be obtained and analysed, performing an automated calculation of the degree of affectation in said ultrasound scans. The ultrasound machine of the invention comprises at least one ultrasonic wave emitting system; an ultrasonic wave detector system, intended to receive the ultrasonic waves emitted by the emitter after impacting with a subject, thus generating ultrasonic images; and a processing unit, connected to the ultrasonic wave receiving system to receive the ultrasonic images and configured to carry out the method for the automated calculation of the degree of affectation described above. The emitting system may comprise trigger electronics and a probe that converts the electrical signal into ultrasonic waves. The receiving system, for its part, may comprise a probe that converts the received ultrasonic echo into an electrical and electronic signal for image reception (amplification, filtering, etc.), processing and representation.

The impact on the capacity of the health system to manage COVID-19 patients, for example, would be very positive, since it is a very specific tool for assessing the lung condition at all stages of the disease, including potential chronic problems in the medium and long term. In addition, it would be useful for the diagnosis and management of patients with other potentially serious lung diseases in certain groups (paediatric patients, pregnant women, etc.).

Preferably, the ultrasound machine of the invention further comprises a display unit, connected to the processing unit and intended to receive and display the combination of the condensed representation of the condition of the pleura with the original image generated by the processing unit.

The specific objectives of the method and the ultrasound machine are:
- Extending the use of lung ultrasound scans to all levels of care and, in particular, to primary care services. For this, it is necessary to reduce the access barrier to the technique, which currently lies in the correct application of the protocol, the interpretation of the images and the determination of the degree of lung affectation. Performing the described method makes it possible to reduce this barrier and extend the technique to a greater number of services, with a faster learning curve.
- Reducing the examination time and simplifying the performance thereof with two objectives: maximising the number of examinations performed in conditions of high healthcare pressure, and minimising the risk of contagion of the examiner, thanks to a shorter exposure time and a simpler protocol, which further minimises the possibility of making self-protection mistakes.
- Generalising the recording of the images, with the aim of facilitating patient follow-up and generating databases that can be used for studying the disease and developing the technique itself.

Some of the main applications of the invention include primary care, hospital emergencies, hospital admission, intensive care units and other pathologies and research and development.

### Primary care:

An important criterion for deciding the hospital admission of a patient with symptoms compatible with COVID-19 is the degree of lung affectation, since pneumonia is one of the main serious complications and its prognosis improves with early treatment. Therefore, having lung ultrasound scans as a diagnostic tool in the early stages of the disease is essential to improving patient management. In particular, it will make it possible to decide with better criterion between home confinement or hospital admission for patients with mild symptoms, based on the real risk of imminently developing pneumonia.

In this sense, the smart assistance tools provided with the method of the invention enable the lung ultrasound scan to be quickly extended to primary care centres, notably reducing the learning curve for managing the equipment and interpreting the images with respect to conventional ultrasound machines.

### Hospital emergencies:

Thoracic ultrasound scans, specifically lung ultrasound scans, are a practice that is increasingly present in hospital emergency services, but also in outpatient services. It is not only more accessible, but also its precision can be similar or even greater than computed tomography (CT) in certain pathologies, without the damage of ionising radiation. However, professionals with experience in lung ultrasound scans are relatively few, and, for example, in the event of pandemics they are under great pressure.

The advantages in this field of application are the simplicity of managing and assessing the images, which reduces the examination time (even for expert personnel), reducing work pressure and the probability of contagion for this group.

Moreover, it also makes it possible to rapidly extend the use thereof to professionals with less experience in emergency services, increasing their capacity to perform this test.

### Hospital admission:

Lung ultrasound scans performed at the patient's bedside are a very valuable tool for patient follow-up, for example in the case of COVID-19. This technique offers very specific information for disease follow-up, can be completed in minutes, and is safe (it does not use ionising radiation). The presence of thickened pleural lines, subpleural consolidations, and B lines are very specific for interstitial syndrome and make it possible to assess the development of pneumonia and the efficacy of the treatment.

Also in this area, its ease of use will make it possible to quickly train more healthcare professionals in lung ultrasound scans and improve patient follow-up due to the definition of examination protocols and data auto-save.

### Intensive care units:

Computed tomography (CT) is the imaging method of reference for assessing lung affectation in patients with COVID-19. However, it has multiple contraindications, whether relative, such as its use in the pregnant or infant population, or absolute, in unstable patients, generally admitted to intensive units, since it requires their transfer to the CT scanner. Therefore, also in intensive care units, lung ultrasound scans at the patient's bedside are very useful for their management, enabling an efficient and continuous assessment of the level of affectation of the lungs.

This is even more critical if the ability to perform CT is exceeded by the number of patients who require it, given that the meticulous decontamination protocols can render it unusable for at least 2 hours.

In this context, the ability of the ultrasound machine of the invention to define and assess the images in less time and generate a graphic record of lung affectation will facilitate patient follow-up, being able to review cases centrally and not only at the patient's bedside as is done today.

### Other pathologies:

Pneumonia is one of the main causes of morbimortality throughout the world. It is also one of the main reasons for seeking emergency services (4.5 million visits in the USA per year) and reasons for hospital admission (2nd cause). This is even more evident among the pediatric population, producing 800,000 deaths among children under the age of 19 year worldwide (31.1 per 100,000 inhabitants), only behind neonatal complications.

Venous thromboembolic disease is the third most common cause of cardiovascular death after myocardial infarction and strokes. In Europe, it is estimated that it causes around 300,000 deaths annually, being the second leading cause of inpatient mortality.

Heart failure is one of the main health problems today. The global magnitude of the problem is difficult to define, in part due to the lack of means and tools for diagnosing it. There are an estimated 23 million people with heart failure worldwide.

These diseases are examples of how performing a lung ultrasound scan can guide the diagnosis and management thereof, having a real impact on the prognosis of the disease.

Therefore, it is expected that an ultrasound machine such as the one proposed in this project will have a positive impact on the national health system beyond the current pandemic. This would not only save costs in performing other ionising tests, such as chest radiography, with less sensitivity than the ultrasound scan, but it would also speed up care times. All this would have repercussions on more efficient attention and, therefore, on a better prognosis.

### Research and development:

At present, lung ultrasounds scans are rarely stored or incorporated into hospital databases, as they are bedside examinations interpreted by on-site professionals. The lack of images and reference databases is a serious limitation both for the development of new technologies and for the study of diseases. The definition of individualised examination protocols and the possibility of automatic graphic records of lung affectation without the intervention of the examiner will have a very positive impact on the generation of databases for disease research and the development of new tools.

### DESCRIPTION OF THE DRAWINGS

As a complement to the description provided and for the purpose of helping to make the features of the invention more readily understandable, in accordance with a practical preferred exemplary embodiment thereof, said description is accompanied by a set of drawings which, by way of illustration and not limitation, represent the following:
Figure 1 shows a schematic representation of the ultrasound machine of the invention.
Figure 2 shows a diagram of a preferred embodiment of the method of the invention.
Figure 3 shows a representation of an example of the graphic records generated by the method, on the left, the overlay of the detected indications on the original image and, on the right, the condensed representation of the pleura, with time on the vertical axis and the detected indications overlaid in grey scale and with patterns.

### PREFERRED EMBODIMENT OF THE INVENTION

The invention relates to a method for automatically identifying and quantifying in real time the artifacts present in lung images, associated with pneumonia and/or dyspnoea, especially in patients with COVID-19 (B Lines, pleural thickening, etc.).

The amount and type of artifacts detected, taking into account all the areas assessed, is used to automatically calculate a degree of affectation of the lungs. Additionally, it provides the option of highlighting artifacts in real time by means of indications overlaid on the image, to assist less experienced examiners in identifying ultrasound findings.

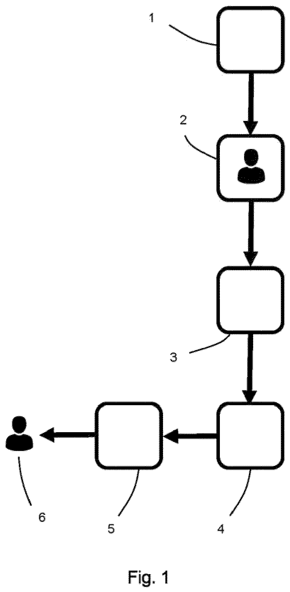

Figure 1 shows a schematic representation of the ultrasound machine of the invention in a preferred embodiment, which comprises:
- An ultrasonic wave emitting system (1).
- An ultrasonic wave receiving system (3), intended to receive the ultrasonic waves emitted by the emitter (1) after impacting with a subject (2), to generate ultrasonic images.
- A processing unit (4), connected to the ultrasonic wave detector (3) to receive the ultrasonic images and configured to carry out a method for the automated calculation of the degree of affectation.
- A display unit (5), connected to the processing unit (4) and intended to receive and display to an examiner (6) a combination of a condensed representation of the condition of the pleura with the original image, generated by the processing unit (4).

In a preferred embodiment of the invention, the emitting system comprises trigger electronics and a probe that converts the electrical signal into ultrasonic waves, while the receiving system comprises a probe that converts the received ultrasonic echo into an electrical and electronic signal for image reception (amplification, filtering, etc.) and representation.

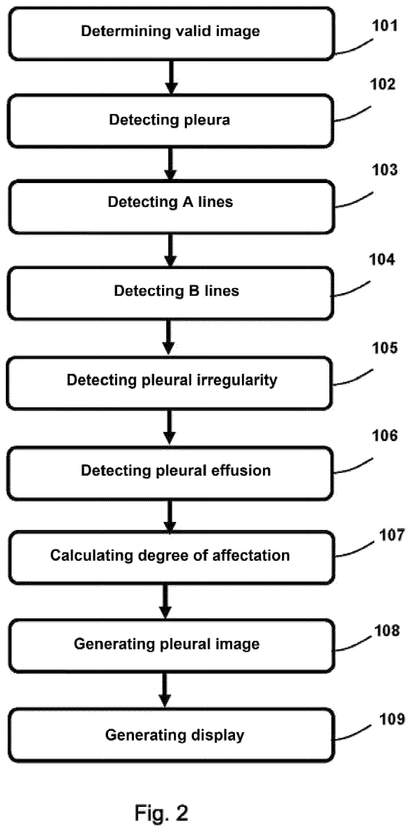

Figure 2 shows a preferred embodiment of the method of the invention consisting of a method with the following steps:
- Determining whether the image is valid or not (101) for processing. It is carried out by means of an analysis of the difference between consecutive images, which in the region above the pleura has to be lower than a threshold for it to be considered that the probe was immobile and that the image can be assessed.
- Detection of the pleura (102): the difference between consecutive images is analysed to reliably detect the boundary between the muscle tissue and the lung parenchyma, without depending on the echogenicity of the pleura in the original image. This step is based on the fact that during a breathing cycle, the region of the image above the pleura hardly changes, whereas the region below the pleura shows great variability. In this case, the average of a set of differential images and a series of filters are used to find the pleura and determine the region to be assessed. Thus, a subtraction of consecutive images and a high-pass filter are used to detect the distance at which the pleura is located, which coincides with the position of the maximum at the outlet of said filter.
- Detecting A lines (103): A lines appear as a replica of the pleura at approximately twice the depth in the image. To obtain them, the maximum grey value in a region located at twice the distance between the transducer and the pleura is calculated for each line in the image. Said maximum is compared with the mean value and the standard deviation of the grey level in the same region, such that an A line is considered to exist when the quotient between the maximum and the standard deviation is greater than a predefined threshold between 5 and 10. Finally, the vertical distance between the points detected in consecutive lines is calculated and the A line is determined as the set of consecutive points, the distance of which is less than a pre-established threshold between 3 and 5 millimetres.
- Detecting B lines (104): a linear regression is applied in the amplitude-depth function of each line in the image, from which the value of the slope is extracted. This parameter is compared with an expected slope due to an attenuation of the medium, defined as signal loss in decibels per centimetre and per megahertz, and it enables discriminating between normal lines (high attenuation) and lines suspected of being B lines (low attenuation). This step can be performed in parallel with the previous one.
- Detecting pleural irregularity (105): measuring the width of the echo generated by the pleura, i.e., measuring the distance between 3 to 12 decibel drop points of the signal around the pleura and comparing it with a pre-established threshold. In a normal lung, the width of the echo is of the order of 1.5 times the period of the ultrasonic signal, whereas, in a pathological lung, it can be up to 4 times greater. Pleural irregularity by itself is not pathological, but it is in the presence of B lines. This step can be performed in parallel with the previous two.
- Detecting a pleural effusion (106): for each line in the image, finding the peak located immediately above the pleura the value of which exceeds a predetermined threshold between 75% and 100% of the grey level of the pleura. Calculating the mean value of the image between this peak and the pleura, and comparing it with a second threshold between 25% and 50% of the grey level of the pleura. If the mean value is less than the threshold, it is a pleural effusion.
- Calculating the degree of affectation (107): the result of the three previous phases is used to calculate a factor of lung affectation, such as the percentage of lines in the image affected by B lines, pleural irregularity or pleural effusion with respect to the total number of lines in the image that pass through the pleura. In this case, two-dimensional filters are used to eliminate noise and reduce false positives.
- Generating the pleural image (108): generating a new form of display by calculating the mean value below the pleura for each line in the image, and constructing a new condensed representation from the mean values on an axis and the time on the second axis. Thus, the acquisition time is placed on the vertical axis, the horizontal position of each line on the horizontal axis, and the mean value of the amplitude coded in grey scale. In this way, it is possible to condense in a single image all the relevant information with respect to the pleura, which can be obtained from a video. This makes it possible to interpret the condition of the pleura very easily and even significantly reduce the volume of data to be saved or analysed for patient follow-up from a single image, avoiding having to analyse an entire video.
- Display (109): combining the results of the previous steps with the original image, to generate two graphic representations:
   a) Overprinting visual coloured aids of the A lines, B lines, pleural irregularities and effusions that were found on the classic grey scale ultrasound image.
   b) Pleural grey scale image, i.e., the condensed representation, with coloured overlay of the regions affected by A lines, B lines, and pleural irregularity.

In one embodiment, the method is implemented on a specialised ultrasound machine or hardware, which receives as input a sequence of images in real time, and deploys said images on the screen together with the result of the automatic detection of artifacts and the degree of lung affectation.

In another embodiment, the method is performed on a computer, operating on a video sequence previously saved by an independent ultrasound machine and generating a processed video.

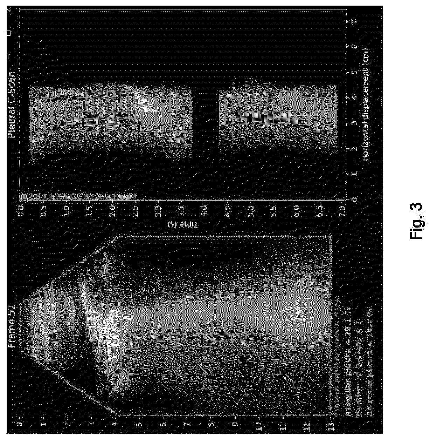

On the left, Figure 3 shows, as a preliminary result, an example of an image wherein the pleura, an A-type line and two B-type lines are automatically detected, which are marked on the original ultrasound scan image. On the right, it shows the condensed image of the pleura, the vertical axis of which is the acquisition time, and which condenses all the information in the video.

## Claims

1. A method for the automated assessment of lung ultrasound scans, comprising a step of applying a method for analysing a set of images of the thoracic cavity, obtained by means of an ultrasound machine, comprising the phases of:
- detecting the pleura (102), as a line that separates an upper area, which has low variability between the set of images and corresponds to the muscle tissue, and a lower area, which has high variability between the set of images and corresponds to the lung parenchyma;
- detecting A lines (103), as lines with a morphology similar to the pleura located at twice the depth in the set of images, which are obtained by calculating the maximum value of the grey level of each line at twice the distance between the transducer and the pleura, and calculating the vertical distance between points detected in consecutive lines, the set of consecutive points, the distance of which is less than a pre-established threshold, less than 10 mm, forming the A line;
- detecting B lines (104), applying a linear regression in the amplitude-depth function of each line in the image, obtaining a slope value for each one, and comparing it with the expected slope due to a nominal attenuation of the medium, defined as signal loss in decibels per centimetre and per megahertz, to identify those with low attenuation as B lines;
- detecting a pleural irregularity (105), measuring the distance between the 6 decibel drop points of the signal around the pleura and comparing it with a pre-established threshold; and
- calculating a degree of affectation (107), such as the percentage of lines in the image affected by B lines, pleural irregularity or pleural effusion with respect to the total number of lines in the image that pass through the pleura.

2. The method according to claim 1, further comprising a step of detecting (106) a pleural effusion, calculating the mean value of the grey level of the image between the pleura and a local maximum located immediately above the pleura, and comparing it with a pre-established threshold between 25% and 50% of the grey level value of the pleura.

3. The method according to any of the preceding claims, further comprising a previous step of classifying (101) images obtained as valid or invalid, by means of a method for analysing the set of images of the thoracic cavity that determines the variability between images obtained consecutively in an upper area, located above the pleura, such that if said variability is less than a pre-established threshold of less than 50%, the images are considered valid.

4. The method according to any of the preceding claims, further comprising a step of generating (108) a condensed representation of the pleura by calculating, for each line in the image, the mean value of amplitude below the pleura and constructing the condensed representation by placing the acquisition time on the vertical axis, the horizontal position of each line on the horizontal axis and the mean value of the amplitude coded in grey scale.

5. The method according to any of the preceding claims, further comprising a step of combining (109) the condensed representation of the pleura and the original image with the detected indications, by means of an overlay of A lines, B lines, pleural irregularities and effusions detected on the original image and an overlay of regions affected by A lines, B lines and pleural irregularities or effusions on the condensed representation of the pleura.

6. The method according to any of the preceding claims, wherein the step of detecting the pleura (102) uses a subtraction of consecutive images and a high-pass filter to detect the distance at which the pleura is located, which coincides with the position of the maximum at the outlet of said filter.

7. The method according to any of the preceding claims, wherein the step of detecting A lines (103) is carried out by searching for the maximum grey value of the image in a time window for each line in the image and comparing said maximum with a mean value and a standard deviation in the same time window, such that an A line is considered to exist when the quotient between the maximum and the standard deviation is greater than a predefined threshold between 5 and 10.

8. The method according to any of the preceding claims, wherein in the step of detecting a pleural irregularity (105), the pre-established threshold is greater than 1.5 times a period of the ultrasonic signal used to generate the images.

9. The method according to any of the preceding claims, wherein in the step of calculating a degree of affectation (106), two-dimensional filters are used to eliminate noise and reduce false positives.

10. The method according to any of the preceding claims, wherein the steps of detecting A lines (103), detecting B lines (104) and detecting a pleural irregularity (105) and pleural effusion (106) are performed simultaneously.

11. An ultrasound machine for obtaining and analysing lung ultrasound scans that enable the automated calculation of the degree of affectation in said ultrasound scans, comprising:
- ultrasonic wave emitting system (1);
- ultrasonic wave detector system (3), intended to receive the ultrasonic waves emitted by the emitter (1) after impacting with a subject (2), to generate ultrasonic images; and
- processing unit (4), connected to the ultrasonic wave detector (3) to receive the ultrasonic images and configured to carry out the method for the automated calculation of the degree of affectation according to any of claims 1 to 10.

12. The ultrasound machine according to claim 11, further comprising a display unit (5), connected to the processing unit (4) and intended to receive and display the combination of the condensed representation of the condition of the pleura with the original image generated by the processing unit (4).

13. A computer program adapted to carry out the steps defined in any of claims 1 to 10.

14. A computer-readable storage medium comprising the computer program of claim 13.
